**Europäisches Patentamt**

**European Patent Office**

**Office européen des brevets**

(19)

(11) Numéro de publication : **0 224 538**
**B1**

(12) **FASCICULE DE BREVET EUROPÉEN**

(45) Date de publication du fascicule du brevet :
23.11.89

(51) Int. Cl.⁴ : **B 01 D 13/00, C 12 M   1/12**

(21) Numéro de dépôt : 86903427.2

(22) Date de dépôt : 09.06.86

(86) Numéro de dépôt international :
**PCT/FR 86/00198**

(87) Numéro de publication internationale :
**WO/8607279 (18.12.86 Gazette 86/27)**

(54) **FILTRATION DE MEMBRANE POUR ANALYSE.**

(30) Priorité : 11.06.85 FR 8508992

(43) Date de publication de la demande :
10.06.87 Bulletin 87/24

(45) Mention de la délivrance du brevet :
23.11.89 Bulletin 89/47

(84) Etats contractants désignés :
**BE CH DE FR GB IT LI NL**

(56) Documents cités :
DE–A– 1 949 059
US–A– 2 763 308
US–A– 2 904 857
US–A– 3 448 011
US–A– 4 094 784
Seifen-Öle-Fette-Wachse, Vol. 99, No. 13, 14. Juni
1973, Augsburg (DE). W. Trampedach:"Membrabfil-
tration in der kosmetischen Industrie", Seiten 369-
372, siehe Seiten 371-372:"2. "Mikrobiologische Rou-
tinekontrollen"

(73) Titulaire : **A.D.R.I.A.**
**6, rue de l'Université**
**F-29191 Quimper (FR)**

(72) Inventeur : **BOURGEOIS, Claude**
**12, rue Albert Camus**
**F-29000 Quimper (FR)**
Inventeur : **COLIN, Jean-Yves**
**1, allée Roz Avel**
**F-29000 Quimper (FR)**

(74) Mandataire : **Dubreuil, Annie**
**Cabinet DUBREUIL Le Suffren 23 rue des Peupliers**
**F-56100 Lorient (FR)**

EP 0 224 538 B1

Jouve, 18, rue St-Denis, 75001 Paris, France

## Description

La présente invention concerne un dispositif destiné à filtrer un liquide et à recueillir sur une membrane des éléments en suspension dans ce liquide en vue de leur analyse.

Un exemple d'application de ce procédé et de cet appareil, conformes à l'invention, porte notamment sur l'analyse du lait.

Par exemple, lorsque l'on veut dénombrer dans le lait des spores de Clostridium Tyrobutyricum qui nuisent à la qualité des fromages notamment de type Emmental, on a besoin d'une méthode répondant à de grandes exigences de sensibilité et de rapidité.

Une méthode mise au point par la demanderesse et qui a déjà fait l'objet de publication, consiste à concentrer les spores par filtration du lait sous pression au travers d'une membrane filtrante, et à dénombrer les colonies obtenues sur cette membrane filtrante appliquée sur un milieu gélosé sélectif, après un temps d'incubation suffisant. Ce procédé dans son principe n'est pas original, mais ce qui l'est, ce sont les conditions précises de cette filtration qui permettent de filtrer des volumes de lait très supérieurs à ceux que permettent de filtrer les procédés habituels.

Une difficulté survient au niveau de la mise en place et de la récupération du filtre, ainsi qu'au niveau de la procédure de filtration qui doivent répondre à des critères très stricts de volume filtré, de commodité d'emploi et d'absence de contaminations parasites, qui ne sont pas satisfaits par les procédés existants.

La filtration analytique se pratique habituellement à l'aide de dispositifs fonctionnant par aspiration, garantissant l'absence de contamination du filtre, et d'un emploi facile adapté aux hautes cadences nécessitées par l'analyse.

Dans le cas de liquides peu filtrables, tels que le lait, il est avantageux, pour accroître le volume filtré, de procéder sous pression, mais les appareils de filtration sous pression du commerce, destinés à la stérilisation préparative des liquides, ne sont pas adaptés à la mise en œuvre répétée à haute cadence nécessitée par l'analyse, et ne permettent pas aisément d'assurer l'absence de contamination parasite du filtre.

La présente invention a précisément pour but de décrire un procédé et un matériel permettant d'effectuer des opérations de filtration analytique sous pression dans les meilleures conditions de commodité d'exécution et de récupération des constituants à analyser.

L'invention concerne la filtration du lait telle que décrite ci-dessus à titre d'exemple, mais elle peut s'appliquer également à la filtration d'autres liquides.

Elle concerne plus précisément un appareil destiné à opérer la filtration sous pression sur un filtre ou une membrane filtrante (11), d'un liquide en vue de la récupération sur le dit filtre (11) de microorganismes, caractérisé en ce qu'il comporte un élément dit tiroir (200) constitué d'au moins un porte-filtre (10) présentant un plan incliné (P2) et d'une plaque métallique dite coin (13) comportant un plan incliné (P1) destiné à venir s'appliquer sur le plan incliné (P2), ce coin (13) pouvant sous l'action d'un vérin (14) occuper deux positions à savoir, une position escamotée qui libère l'accès au porte-filtre (10) et une position de coincement assurant une étanchéité au niveau des plans inclinés (P1) et (P2), le filtre (11) que porte le porte-filtre (10) se trouvant alors en contact avec le liquide à filtrer contenu dans un réservoir (3).

L'invention concerne également le procédé d'utilisation d'un tel appareil conformément à l'objet de la revendication 3.

L'invention sera mieux comprise à l'aide de la description de l'appareil et de son fonctionnement qui va suivre, et des figures jointes parmi lesquelles :

la figure 1 représente l'ensemble du dispositif de filtration, le système de blocage du filtre étant en position opérationnelle ;

la figure 2 est une vue en plan et de dessus du porte-filtre ;

la figure 3 montre le système de blocage du filtre et porte-filtre, en position de libération.

Pour plus de clarté, les mêmes éléments portent les mêmes références dans toutes les figures.

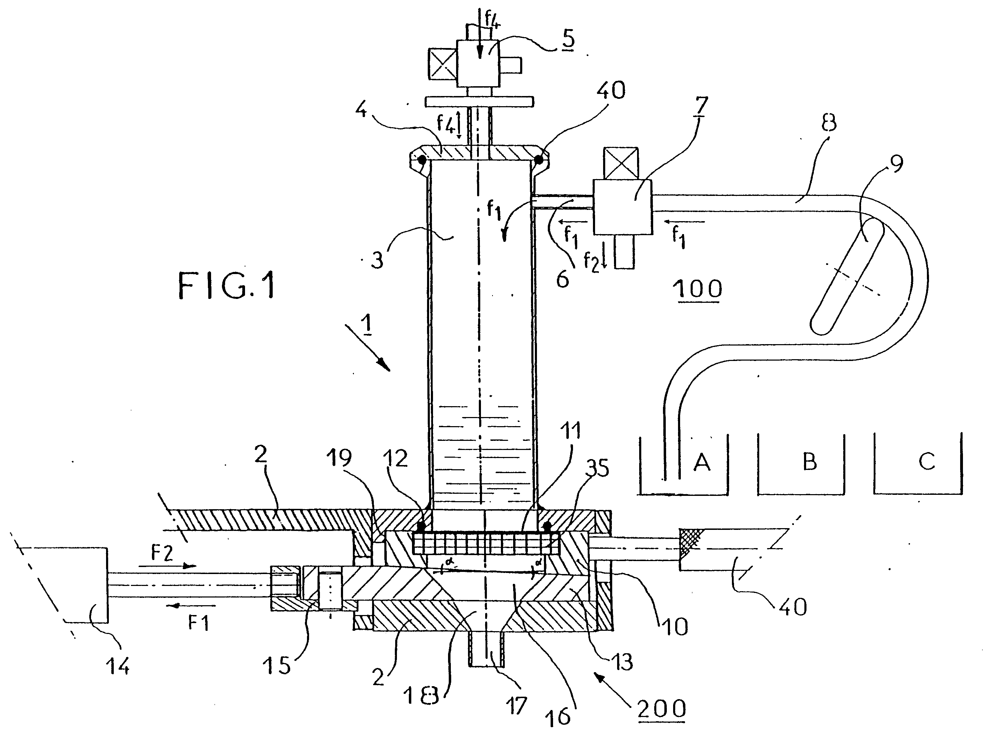

Comme le montrent les figures 1 à 3, cet appareil est caractérisé en ce qu'il est constitué d'un boîtier (2), schématiquement représenté, qui reçoit deux ensembles actifs.

Le premier ensemble actif 100, disposé verticalement sur la partie supérieure de l'appareil, comporte un cylindre 3 faisant office de réservoir du produit à filtrer, avec ses systèmes d'alimentation et pneumatiques associés.

Au sommet de ce réservoir 3 se trouve un couvercle étanche 4 fixé grâce à un joint torique 40, sur lequel est adapté un système pneumatique avec électrovanne 5. Un tube d'alimentation 6, associé à un système d'électrovanne 7, débouche dans le cylindre-réservoir 3, sur une génératrice, et, au sommet de celui-ci. Il permet, par l'intermédiaire d'un tuyau souple 8 et d'une pompe 9 (à palettes par exemple), d'introduire dans le réservoir 3, sans soulever le couvercle étanche 4 du réservoir, le liquide à analyser ainsi que différents liquides d'addition et de rinçage suivant un cycle programmé et adapté au filtrage à effectuer, ces liquides étant prélevés dans les récipients A, B ou C suivant la composition des liquides et les besoins et l'ordre d'introduction de ceux-ci.

Le second ensemble actif 200, appelé tiroir dans la suite de la description, est placé à la partie inférieure de l'appareil, sous le cylindre-réservoir 3.

Il est constitué d'un porte-filtre 10 amovible, muni d'une poignée 40, qui reçoit un filtre 11 qui n'est autre qu'une membrane poreuse sur laquelle les produits de filtration se déposeront et seront recueillis pour l'analyse.

Le filtre 11, sur son porte-filtre 10, est maintenu en contact avec le liquide à filtrer, d'une manière étanche grâce d'une part, à un joint torique 12, et d'autre part, selon une des caractéristiques de l'invention, grâce à un système de coin 13 qui peut s'escamoter sous l'action d'un vérin 14 coopérant avec un système de liaison 15 à plusieurs degrés de liberté, empêchant tout blocage intempestif en cours de fonctionnement. Dans la plaque métallique formant le coin 13, un passage 16 d'évacuation du liquide filtré est aménagé.

Le coincement correct et l'étanchéité du système, hormis l'office du joint torique, est obtenu par la coopération des deux plans inclinés P1 et P2, d'angle (α), respectivement du coin 13 et du porte-filtre 10. Dans l'exemple choisi, l'angle (α) est de l'ordre de 2°.

En reculant le coin 13 sous l'action du vérin 14, selon la flèche F1, on supprime ainsi la pression verticale s'exerçant sur le porte-filtre 10 et son filtre 11 qui peuvent ainsi être récupérés avec les éléments à analyser.

Le liquide est évacué pendant l'opération de filtrage, par l'orifice 17, en s'écoulant à travers le fond du corps 2 de l'appareil par un orifice conique 18 prévu à cet effet.

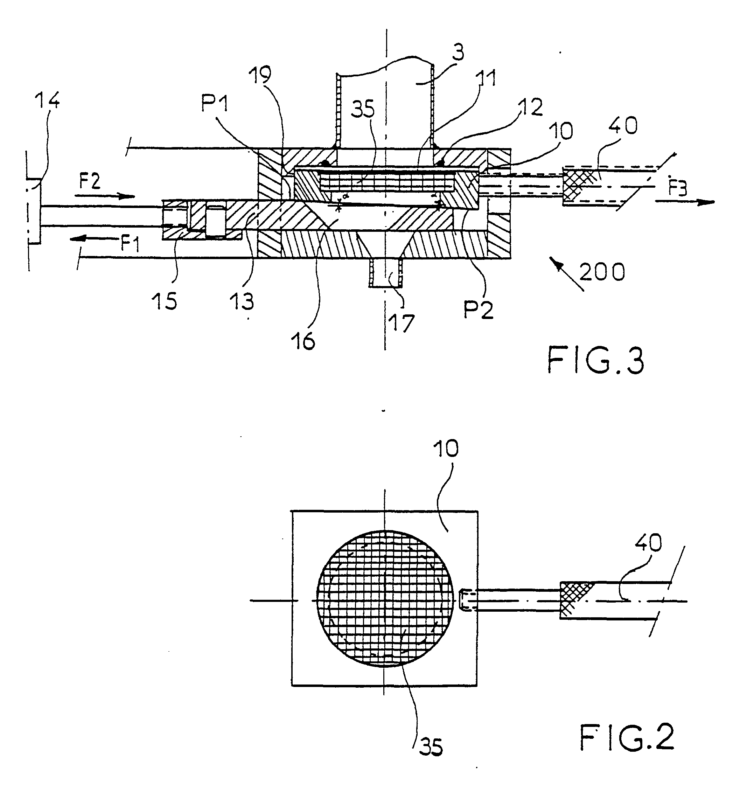

Sur la figure 2, on montre l'existence et la position d'une grille 35 placée dans la cavité du porte-filtre 10, sur laquelle vient s'appuyer le filtre 11 afin de le maintenir plan, sans empêcher l'écoulement du liquide à travers le dispositif.

Une application privilégiée de l'invention, mettant en œuvre un dispositif conforme à l'invention tel que décrit précédemment, fait maintenant l'objet de la suite de la description.

Il s'agit de recueillir des microorganismes, par exemple des Clostridium Tyrobutyricum, en suspension dans du lait, afin de les analyser. On considère le couvercle 4, associé à son système pneumatique 5 en place, ainsi que le système d'alimentation 100 prêt à fonctionner, l'intérieur du dispositif étant propre et stérile.

Le porte-filtre 10 est sorti de son tiroir 200 et prêt à recevoir un filtre circulaire 11. Le coin 13 est en position escamotée. Comme le montre la figure 3, le porte-filtre 10 et son filtre 11 sont introduits dans le tiroir jusqu'à son point de butée 19. Le porte-filtre 10 et son filtre 11 peuvent alors être remontés et bloqués, sous l'action du vérin 14 poussant le coin 13 dans le sens de la flèche F2.

La quantité de lait nécessaire et suffisante, en provenance du récipient A par exemple, est alors introduite dans le réservoir 3 grâce à la pompe 9 et au système d'électrovanne 7 positionné dans le sens passant, suivant la flèche F1.

Le cylindre 3 est ensuite mis sous pression d'air grâce à l'électrovanne 5, positionné dans le sens passant suivant la flèche f4 (dans l'exemple choisi, la pression est de 3 bars). Sous l'effet de la pression, la filtration commence à s'opérer à travers le filtre 11, le liquide s'évacuant par l'orifice 17 et les microorganismes se déposant sur le filtre 11.

Après filtration totale de la quantité de lait introduit dans le réservoir 3, la pression d'air est supprimée par l'action de l'électrovanne 5. Une opération de rinçage à l'eau est alors effectuée, sans besoin d'ouverture du réservoir 3.

Cette eau qui peut se trouver dans le récipient B, est alors introduite dans le réservoir 3 après commutation du tuyau 8 de A dans B, puis évacuée à travers l'orifice 17, suivant le même processus que celui décrit pour l'introduction et l'évacuation du lait.

Après cette opération de rinçage, les microorganismes déposés sur le filtre 11 peuvent être récupérés. Conformément à l'invention et selon une caractéristique importante de l'invention liée notamment à l'architecture du porte-filtre décrit précédemment, cette opération délicate qui ne doit ni arracher, ni polluer le filtre 11 et son dépôt à analyser, est réalisée dans les plus brefs délais et dans des conditions optimales.

Pour récupérer le filtre dans les conditions souhaitées, il suffit en effet de faire reculer le coin 13, sous l'action du vérin 14, suivant la flèche F1, pour libérer et faire descendre le porte-filtre 10 et son filtre 11 d'une distance suffisante pour qu'en tirant, selon F3, et éventuellement en basculant le porte-filtre 10 grâce à la poignée 40, le retrait du porte-filtre puisse s'effectuer sans aucun contact ni frottement du filtre.

Après remise en place du porte-filtre, sans filtre ou avec un filtre de substitution, un nettoyage peut s'effectuer suivant le processus précédemment décrit, en permutant les tuyaux dans un récipient C par un moyen manuel ou automatique et programmé.

L'ensemble d'une opération de filtrage et de nettoyage peut donc être réalisé selon une succession d'étapes qui peuvent être regroupées en 5 cycles principaux décrits ci-dessous.

Cycle A

1) Mise en place du filtre 11 sur le porte-filtre 10.

2) Mise en place du porte-filtre 10 dans le tiroir 200 (le tube 8 est commuté au récipient A contenant le lait à filtrer).

3) Ouverture de l'électrovanne 7 pour l'introduction du lait dans le réservoir 3 suivant la flèche f1, sous l'action de la pompe 9 (l'électrovanne 5 est fermée).

4) Fermeture de l'électrovanne 7 après introduction du lait dans le réservoir 3 en quantité nécessaire et suffisante (dosage en volume préalablement fixé).

5) Ouverture de l'électrovanne 5, mise sous pression de 3 bars (par exemple) suivant la flèche f4, passage du lait à travers le filtre sous l'effet de la pression d'air, et évacuation par l'orifice 17 du liquide filtré.

6) Fermeture de l'électrovanne 5.

Cycle B : Rinçage à l'eau

7) Commutation du tube 8 du récipient A dans le récipient B contenant de l'eau.

8) Ouverture de l'électrovanne 7 et mise en route de la pompe 9.

9) Admission d'eau et remplissage du réservoir 3 par celle-ci.

10) Fermeture de l'électrovanne 7.

11) Ouverture de l'électrovanne 5 amenant de l'air sous pression suivant la flèche F4. Passage de l'eau à travers le filtre sous l'effet de la pression d'air (les microorganismes déposés ne sont pas entraînés). Evacuation de l'eau à travers l'orifice 17.

12) Fermeture de l'électrovanne 5.

Cycle C : Récupération des dépôts à analyser (A ce stade, les électrovannes 5 et 7 sont fermées)

13) Ouverture du tiroir 200 (comme il a été expliqué dans la description du second ensemble actif), et extraction du porte-filtre 10 et de son filtre 11.

14) Récupération du filtre 11 et de son dépôt de microorganismes à analyser.

Cycle D : Nettoyage spécifique du dispositif

15) Introduction d'un filtre sur le porte-filtre 10 (ce filtre de substitution sera perdu).

16) Introduction du porte-filtre 10 dans le tiroir 200.

17) Commutation du tuyau 8 dans le récipient C contenant une solution spécifique de rinçage ou d'aseptisation.

18) Le cycle B, étape (8), peut alors être reproduit jusqu'à la récupération du filtre de substitution qui sera détruit.

Cycle E : Rinçage au lait

Ce cycle consiste à mettre tout le dispositif et sa tuyauterie dans l'atmosphère du lait ou liquide à filtrer.

Il suffit alors de commuter le tuyau 8 dans le récipient A contenant le lait à analyser, et d'effectuer un cycle A à partir de la séquence 2, en ayant placé ou non sur le porte-filtre 10 un filtre de substitution.

Après remise d'un filtre actif 11, les cycles A à C complets pourront alors être effectués pour la récupération des éléments à analyser.

Tous ces cycles peuvent être réalisés autant de fois qu'on le désire sans que l'opérateur soit obligé de procéder à des démontages fastidieux, longs et souvent peu fiables sur le plan du nettoyage.

Les applications d'un tel dispositif sont nombreuses et se retrouvent dans le cas de l'analyse du lait (application décrite à titre d'exemple), mais également dans la technique de fabrication d'un grand nombre de produits tels que la bière par exemple.

**Revendications**

1. Appareil destiné à opérer la filtration sous pression sur un filtre ou une membrane filtrante (11), d'un liquide en vue de la récupération sur le dit filtre (11) de microorganismes, caractérisé en ce qu'il comporte un élément dit tiroir (200) constitué d'au moins un porte-filtre (10) présentant un plan incliné (P2) et d'une plaque métallique dite coin (13) comportant un plan incliné (P1) destiné à venir s'appliquer sur le plan incliné (P2), ce coin (13) pouvant sous l'action d'un vérin (14) occuper deux positions à savoir, une position escamotée qui libère l'accès au porte-filtre (10) et une position de coincement assurant une étanchéité au niveau des plans inclinés (P1) et (P2), le filtre (11) que porte le porte-filtre (10) se trouvant alors en contact avec le liquide à filtrer contenu dans un réservoir (3).

2. Appareil selon la revendication 1, caractérisé en ce que le réservoir (3) est d'une part relié, via un tube (8), à au moins trois récipients (A, B, C) par l'intermédiaire d'un système de pompe (9) et de commutation (7), et d'autre part relié, via une électrovanne (5), à une source d'air sous pression (f4).

3. Utilisation d'un appareil selon la revendication 2 selon un procédé comportant les cycles suivants :

Cycle a :

mise en place du filtre (11) sur le porte-filtre (10) et coincement du porte-filtre par le coin (13) ;

mise en communication du réservoir (3) avec le récipient (A) pour introduction du liquide à filtrer dans le réservoir (3) ;

ouverture de l'électrovanne (5) et mise en place de la pression (f4) dans le réservoir (3) provoquant la filtration, avec évacuation du filtrat par un orifice (17) ;

fermeture de l'électrovanne (5).

Cycle b :

mise en communication du récipient (B) avec le réservoir (3) ;

mise en place de la pression (f4) ;

rinçage du filtre par le liquide du récipient (B) sans entraînement des microorganismes recueillis sur le filtre durant le cycle (a) ;

fermeture de l'électrovanne (5).

Cycle c :

fermeture de l'électrovanne (5) et de la commutation (7) ;

décoincement du coin (13) ;

extraction du filtre (10) et récupération des microorganismes recueillis sur le filtre.

4. Utilisation selon la revendication 3, caractérisé en ce que, après les cycles (a), (b), (c) s'ajoute un cycle (d) consistant à introduire à la place du filtre (11), un filtre de substitution et à mettre en communication le réservoir (3) avec le récipient (C) contenant une solution d'aseptisation et à soumettre le réservoir (3) à la pression d'air (f4), puis à extraire, après ce rinçage, le filtre de substitution avant de recommencer une séquence de filtration (a), (b), (c).

5. Utilisation selon la revendication 4, caractérisé en ce que, après le rinçage par la solution du récipient (C) et avant extraction du filtre de substitution, on met en communication le réservoir (3) avec le récipient (A) contenant le liquide à filtrer et on soumet le réservoir (3) à la pression d'air (f4) en opérant ainsi un rinçage par le liquide à filtrer, avant extraction du filtre de substitution et reprise d'une séquence de filtration (a), (b), (c).

## Claims

1. Apparatus intended to perform the filtration of a liquid under pressure on a filter or filtering membrane (11) for the purpose of recovering microorganisms on the said filter characterized in that it comprises an element referred to as a drawer (200) comprising at least one filter carrier (10) having a sloping plane (P2) and a metal plate referred to as a wedge (13) having a sloping plane (P1) intended to come to bear against the sloping plane (P2), this wedge (13) being able under the action of a jack (14) to assume two positions, namely a withdrawn position which frees access to the filter carrier (10) and a wedging position ensuring a seal at the level of the sloping planes (P1) and (P2), the filter (11) which the filter carrier (10) carries then being in contact with the liquid to be filtered contained within a tank (3).

2. Apparatus according to claim 1, characterized in that the tank (3) is on the one hand connected via a tube (8), to at least three vessels (A, B, C) through a pump (9) and switching (7) system and on the other hand, connected via an electro valve (5), to a source of air under pressure (f4).

3. Use of an apparatus according to claim 2 in accordance with a process comprising the following cycles :

Cycle a :

positioning the filter (11) on the filter carrier (10) and wedging the filter carrier by means of the wedge (13) ;
putting the tank (3) in communication with the vessel (A) for introduction into the tank (3) of the liquid to be filtered ;
opening the electro valve (5) and establishing the pressure (f4) within the tank (3) causing the filtration with discharge of the filtrate through an orifice (17).

Cycle b :

closing the electromagnetic valve (5) ;
putting the vessel (B) in communication with the tank (3) ;
establishing the pressure (f4) ;
flushing the filter by means of the liquid from the vessel (B) without washing away the microorganisms collected on the filter during the cycle (a) ;
closing the electro valve (5).

Cycle c :

closing the electro valve (5) and the switch (7) ;
freeing the wedge (13) ;
withdrawing the filter (10) and recovering the micro-organisms collected on the filter.

4. Use according to claim 3, characterized in that after the cycle (a), (b) and (c) there is added a cycle (d) consisting in inserting a substitute filter in place of the filter (11) and in putting the tank (3) in communication with the vessel (C) containing an asepticising solution and in subjecting the tank (3) to the air pressure (f4), then removing the substitute filter, after this flushing before restarting a filtering sequence (a), (b), (c).

5. Use according to claim 4, characterized in that after the flushing by means of the solution from the vessel (C) and before the withdrawal of the substitute filter, the tank (3) is put in communication with the vessel (A) containing the liquid to be filtered and the tank (3) is subjected to the air pressure (f4), thereby performing a flushing action by means of the liquid to be filtered, prior to withdrawal of the substitute filter and resumption of a filtering sequence (a), (b), (c).

## Patentansprüche

1. Gerät, dazu bestimmt, die unter Druck erfolgende Filtrierung einer Flüssigkeit auf einem Filter oder einer Filtriermembrane (11) zwecks Gewinnung von Mikroorganismen auf diesem Filter (11) zu bewirken, dadurch gekennzeichnet, daß es ein Schieberelement (200) umfaßt, bestehend aus mindestens einem Filterhalter (10) mit einer schrägen Ebene (P2), sowie aus einer Keil (13) genannten Metallplatte, die eine schiefe Ebene (P1) aufweist, welche dazu bestimmt ist, gegen die schiefe Ebene (P2) anzuliegen, wobei dieser Keil (13) durch Einwirkung eines Arbeitszylinders (14) zwei Stellungen einnehmen kann, nämlich eine fortbewegte Stellung, die den Zugang zum Filterhalter (10) freigibt, sowie eine Verkeilungsstellung, die für eine Dichtigkeit an den schiefen Ebenen (P1) und (P2) sorgt, wobei sich der Filter (11), den der Filterhalter (10) trägt, in Kontakt mit der in einem Tank (3) enthaltenen zu filtrierenden Flüssigkeit befindet.

2. Gerät nach Anspruch 1, dadurch gekennzeichnet, daß der Tank (3) einerseits über einen Schlauch (8) mit mindestens drei Behältern (A, B, C) über ein Pump- (9) und Schaltsystem (7) verbunden ist, und andererseits über ein Magnetventil (5) mit einer Druckluftquelle (f4).

3. Verwendung eines Geräts nach Anspruch 2 nach einem die folgenden Zyklen umfassenden Verfahren :

Zyklus a :

Einsetzen des Filters (11) auf den Filterhalter (10) und Festklemmen des Filterhalters mit Keil (13) ;

Herstellung der Verbindung des Tanks (3) mit dem Behälter (A) für das Einführen der zu filtrierenden Flüssigkeit in den Tank (3) ;

Öffnen des Magnetventils (5) und Aufbau des Drucks (f4) im Tank (3), wodurch die Filtrierung bewirkt wird, mit Abführen des Filtrats durch eine Öffnung (17).

Schließen des Magnetventils (5).

Zyklus b :

Herstellung der Verbindung des Behälters (B) mit dem Tank (3) ;

Aufbau des Drucks (f4) ;

Spülung des Filters mit der Flüssigkeit des Behälters (B) ohne Mitziehen der während des Zyklus (a) auf dem Filter angesammelten Mikroorganismen ;

Schließen des Magnetventils (5).

Zyklus c :

Schließen des Magnetventils (5) und der Schaltung (7) ;

Lösen der Keils (13) ;

Herausziehen des Filters (10) und Gewinnung der auf dem Filter angesammelten Mikroorganismen.

4. Verwendung nach Anspruch 3, dadurch gekennzeichnet, daß sich an die Zyklen (a), (b), (c) ein Zyklus (d) anschließt, der darin besteht, anstelle des Filters (11) einen Filterersatz einzulegen, und den Tank (3) mit dem eine Desinfektionslösung enthaltenden Behälter (C) in Verbindung zu setzen und den Tank (3) dem Luftdruck (f4) auszusetzen, dann nach dieser Spülung den Filterersatz herauszuziehen, bevor ein Filtrierungsablauf (a), (b), (c) neubegonnen wird.

5. Verwendung nach Anspruch 4, dadurch gekennzeichnet, daß man nach Spülung mit der Lösung des Behälters (C) und vor Herausziehen des Filtersersatzes den Tank (3) mit dem die zu filtrierende Flüssigkeit enthaltenden Behälter (A) in Verbindung setzt und den Tank (3) dem Luftdruck (f4) aussetzt, wobei auf diese Weise eine Spülung mittels der zu filtrierenden Flüssigkeit vor Herausziehen des Filtersersatzes und Neubeginn eines Filtrierungsablaufs (a), (b), (c) bewirkt wird.

FIG.1

$f_4$

5

4

40

$f_4$

7

8

9

$f_1$

3

$f_1$

6

$f_1$

$f_2$

100

1

11

2

19

12

35

A

B

C

$\alpha$

$\alpha$

F2

10

14

F1

15

2

18

17

16

13

40

200

EP 0 224 538 B1

FIG.3

FIG.2